# FASCICULE DE BREVET EUROPEEN

(11) **EP 3 921 192 B1**
(45) Date de publication et mention de la délivrance du brevet: **07.06.2023**
(21) Numéro de dépôt: 20702480.3
(22) Date de dépôt: 05.02.2020
(51) Int. Cl.: B60H 1/00, B60H 1/22, B60H 3/00, B60H 3/06, A61L 2/04, A61L 9/013

(54) **SYSTEME ET PROCEDE DE NETTOYAGE D'UN HABITACLE DE VEHICULE**
SYSTEM UND VERFAHREN ZUR REINIGUNG EINES FAHRZEUGINNENRAUMES
SYSTEM AND METHOD FOR CLEANING A VEHICLE PASSENGER COMPARTMENT

(30) Priorité: 07.02.2019 FR 1901215
(43) Date de publication de la demande: 15.12.2021
(73) Titulaire: ELLONA, 31400 Toulouse (FR)
(72) Inventeur: MIFSUD, Jean-Christophe, 82400 Goudourville (FR)
(74) Mandataire: Argyma
(86) Numéro de dépôt international: PCT/EP2020/052822
(87) Numéro de publication internationale: WO 2020/161168

(56) Documents cités:
- EP-A1- 3 278 817
- WO-A2-2008/006035
- FR-A1- 3 055 585
- JP-A- H0 958 258
- US-A1- 2014 271 347

## Description

### DOMAINE TECHNIQUE DE L'INVENTION

La présente invention concerne le domaine du nettoyage d'un habitacle de véhicule et vise plus particulièrement un système et un procédé de nettoyage de l'air ambiant et des éléments de l'habitacle dudit véhicule.

### ETAT DE LA TECHNIQUE

De manière connue, un véhicule automobile permet de déplacer ses utilisateurs d'un lieu donné à un autre. Un véhicule comprend un habitacle qui est défini comme la partie du véhicule réservée aux utilisateurs. Cet habitacle comprend divers éléments d'habitacle pour assurer le confort et la sécurité des utilisateurs (siège, pare-soleil, etc.). Au cours d'un déplacement, l'habitacle est souillé par diverses impuretés telles que les odeurs de sueur, de cigarette ou de nourriture de ses utilisateurs, les odeurs des animaux de compagnie ou encore les composés organiques volatils, connues de l'homme du métier sous l'abréviation « COV », comme des particules polluantes, de la poussière ou du pollen provenant de l'extérieur. Toutes ces impuretés imprègnent aussi bien l'air ambiant de l'habitacle que les éléments de l'habitacle et nuisent au confort des utilisateurs du véhicule. Cela est particulièrement nuisible pour les véhicules automobiles soumis à un taux d'occupation élevé, à savoir les véhicules collectifs (bus, etc.) et les véhicules en partage (voiture autonome, voiture de location, etc.).

Actuellement, pour nettoyer l'habitacle d'un véhicule ayant un taux d'occupation élevé, le véhicule est déplacé périodiquement vers une station de nettoyage où les impuretés de l'habitacle sont évacuées au moyen d'un appareil de nettoyage externe, comme un aspirateur par exemple. Ce procédé présente l'inconvénient d'être onéreux étant donné qu'il nécessite, d'une part, le déplacement du véhicule vers une station dédiée au nettoyage et, d'autre part, de la main d'oeuvre pour réaliser le nettoyage. De surcroît, un tel nettoyage est complexe à planifier car l'habitacle n'est pas souillé de la même façon suivant la fréquence d'utilisation et le taux d'occupation du véhicule. Or, si un nettoyage anticipé est un gaspillage de temps et d'argent, un nettoyage tardif peut provoquer le mécontentement des utilisateurs du véhicule.

On connaît dans l'art antérieur, par la demande de brevet EP3278817A1, un procédé de nettoyage de l'habitacle d'un véhicule soumis à un taux d'occupation élevé qui enseigne d'injecter de l'air chaud combiné à un produit désinfectant dans l'habitacle clos du véhicule au moyen d'un conduit provenant d'un système extérieur. Ce procédé présente l'avantage de ne pas nécessiter de main d'oeuvre pour réaliser le nettoyage mais ne permet pas de résoudre les autres inconvénients cités dans le paragraphe précédent (déplacement nécessaire du véhicule et planification complexe du nettoyage). Ce procédé présente en outre l'inconvénient d'évacuer uniquement les impuretés présentes dans l'air de l'habitacle.

De manière alternative, on connaît par la demande de brevet US2018/0126960A1 un procédé de nettoyage de l'habitacle d'un véhicule autonome dans lequel ledit véhicule autonome est déplacé vers une station de nettoyage lorsque des capteurs de niveau d'insalubrité présents dans l'habitacle l'indiquent. Ce procédé présente l'avantage de pouvoir planifier avec précision un nettoyage du véhicule mais ne permet pas de résoudre les autres inconvénients (déplacement nécessaire du véhicule, utilisation de main d'oeuvre pour réaliser le nettoyage, etc.). On connait également par la demande de brevet US201/271347A1 un système de décontamination de véhicule, en particulier du compartiment d'un aéronef, configuré pour éliminer ou inactiver tout pathogène à bord du véhicule, tel qu'un virus, un germe, etc.

Il existe donc un besoin pour un système et un procédé de nettoyage de l'habitacle d'un véhicule soumis à un taux d'occupation élevé, par exemple un véhicule autonome, qui soit peu onéreux, qui puisse être effectué dès lors que l'état de propreté dudit véhicule n'est pas satisfaisant et qui traite efficacement les impuretés.

Bien que l'invention soit née à l'origine pour un véhicule automobile, elle s'applique plus généralement à tout véhicule comportant un habitacle, en particulier, un train, un avion, un bateau, etc.

### PRESENTATION DE L'INVENTION

A cet effet, l'invention concerne un véhicule comprenant un habitacle et un système de nettoyage de l'habitacle comprenant au moins un organe de mesure du niveau d'insalubrité N dudit habitacle, au moins un organe de chauffage configuré pour chauffer l'air ambiant de l'habitacle à une température supérieure à 40°C afin de désorber les impuretés de l'habitacle, au moins un organe de ventilation configuré pour évacuer l'air ambiant et les impuretés désorbées à l'extérieur de l'habitacle et au moins un organe de commande configuré pour activer l'organe de chauffage si le niveau d'insalubrité N mesuré est supérieur à un niveau d'insalubrité maximal prédéterminé N_{ET}.

L'invention est remarquable en ce que le système de nettoyage est interne au véhicule, ce qui évite de déplacer le véhicule vers une station de nettoyage, et permet de purifier l'habitacle de façon autonome sans main d'oeuvre. Ce système de nettoyage est de plus efficace, car étant compris dans l'habitacle, il est au plus près des impuretés à traiter. Cette position permet également avantageusement de faciliter l'installation et la maintenance du système de nettoyage. Cela est particulièrement avantageux dans le cas de véhicules partagés.

Selon un aspect de l'invention, l'organe de chauffage est configuré pour chauffer l'air ambiant de l'habitacle à une température supérieure à 45°C de préférence à 50°C. De manière avantageuse, la majorité des impuretés présentes dans l'habitacle sont désorbées à cette température, c'est-à-dire qu'elles sont extraites des éléments de l'habitacle et passent dans une phase vapeur, ce qui facilite leur extraction par ventilation.

Selon un autre aspect de l'invention, l'organe de chauffage est configuré pour chauffer l'air ambiant de l'habitacle à une température inférieure à 80°C, de sorte à ne pas endommager les éléments dudit habitacle.

De manière préférée, le véhicule comprend au moins un élément d'habitacle comprenant au moins un organe de chauffage configuré pour chauffer l'élément d'habitacle à une température supérieure à 40°C afin de former un élément d'habitacle chauffant pour désorber les impuretés de l'élément d'habitacle. De manière avantageuse, le système de nettoyage permet non seulement de nettoyer l'air ambiant de l'habitacle mais également les éléments d'habitacle, afin d'éliminer les impuretés captées de l'élément d'habitacle et qui sont stockées en son sein.

Selon un aspect de l'invention, l'organe de chauffage est intégré à l'élément d'habitacle, de sorte à pouvoir nettoyer efficacement de manière interne ledit élément d'habitacle et non de manière superficielle. En outre, lorsque l'organe de chauffage est intégré, l'aspect extérieur de l'élément n'est pas modifié, ce qui est avantageux sur le plan du confort et de l'esthétique.

Selon un autre aspect de l'invention, l'organe de chauffage est positionné en surface de l'élément d'habitacle, de sorte à pouvoir nettoyer efficacement la partie superficielle dudit élément d'habitacle, particulièrement exposée aux sources d'impuretés. Ces deux configurations d'installation permettent avantageusement un nettoyage optimal de l'habitacle en fonction du type d'élément d'habitacle.

De manière préférée, l'organe de chauffage est intégré ou positionné en surface d'un élément d'habitacle comprenant de la mousse polyuréthane ou du polychlorure de vinyle ou de l'acrylonitrile butadiène styrène ou du polypropylène ou du polyéthylène ou du polyester ou un alliage de ces thermoplastiques, de sorte à améliorer le chauffage et la désorption. De préférence, l'organe de chauffage est intégré dans le volume de l'élément d'habitacle.

Préférentiellement, au moins un élément d'habitacle chauffant est un siège, afin de nettoyer les impuretés provenant principalement des vêtements des utilisateurs, comme de la sueur.

De manière préférée, le véhicule comprend une pluralité d'éléments d'habitacle chauffants pour un nettoyage global de l'habitacle.

Préférentiellement, au moins un élément d'habitacle chauffant est le revêtement de plancher, afin de nettoyer les impuretés provenant principalement des chaussures des utilisateurs, comme de la terre.

Préférentiellement, au moins un élément d'habitacle chauffant est le revêtement de pavillon, afin de nettoyer des impuretés telles que des COV et des odeurs, de fumée de cigarette ou de nourriture par exemple.

Préférentiellement, au moins un élément d'habitacle chauffant est le revêtement de parois latérales, afin de nettoyer les impuretés provenant principalement des utilisateurs ou encore des toxiques lourds.

Préférentiellement, au moins un élément d'habitacle chauffant est un pare-soleil, afin de nettoyer les impuretés provenant principalement des utilisateurs, du véhicule ou de la pollution extérieure.

Préférentiellement, au moins un élément d'habitacle chauffant est la planche de bord, afin de nettoyer les impuretés provenant principalement des utilisateurs, du véhicule ou de la pollution extérieure.

Préférentiellement, au moins un élément d'habitacle chauffant est le revêtement d'une portière, afin de nettoyer les impuretés provenant principalement des utilisateurs, du véhicule ou de la pollution extérieure.

De préférence, l'organe de chauffage se présente sous la forme d'une résistance chauffante en forme de serpentin. De préférence, l'organe de chauffage est alimenté de manière filaire ou sans fil, par exemple, par induction. Un organe de chauffage sous une telle forme permet de chauffer de façon homogène et efficace son voisinage et donc de nettoyer de façon globale et non partielle ce voisinage. Un organe de chauffage sans fil permet une plus grande liberté de positionnement dans un élément d'habitacle qui est alors amovible dudit habitacle.

Selon un aspect de l'invention, l'organe de mesure se présente sous la forme d'un capteur de gaz, tel qu'un capteur à oxyde métallique, et relève le niveau d'insalubrité N via le relevé de la présence de toxiques volatils tels que le dioxyde d'azote N₁ et/ou le benzène toluène xylène N₂ et/ou l'ozone N₃ et/ou le formaldéhyde N₄ et/ou tous autres toxiques volatils, tels que des COV. Ce relevé d'une pluralité de données N₁-N₄ permet une mesure fiable et objective du niveau d'insalubrité N.

Préférentiellement, le niveau d'insalubrité maximal prédéterminé N_{ET} est obtenu à partir de banques de données sur la présence maximale autorisée de toxiques volatils dans l'habitacle du véhicule. De manière avantageuse, le niveau d'insalubrité maximal prédéterminé N_{ET} est donc un moyen de comparaison pertinent car basé sur le type d'impuretés que l'on retrouve dans le véhicule.

De manière préférée, la puissance de l'organe de ventilation est adaptée au niveau d'insalubrité N et au volume de l'habitacle de sorte à évacuer rapidement les impuretés en suspension dans l'air ambiant et à minimiser la consommation d'énergie.

Selon un aspect de l'invention, le véhicule comprend au moins un dispositif de charge électrique tel qu'une batterie électrique, de sorte que l'organe de mesure, l'organe de commande, l'organe de chauffage et l'organe de ventilation sont alimentés électriquement par un système interne au véhicule, ledit dispositif de charge électrique fonctionnant indépendamment de l'état en marche ou à l'arrêt du véhicule.

De manière préférée, le système de nettoyage comprend au moins un organe de filtrage configuré pour traiter les impuretés désorbées lors de leur évacuation vers l'extérieur de l'habitacle. De manière avantageuse, ce système de nettoyage ne rejette pas d'impuretés potentiellement nocives dans l'atmosphère.

Préférentiellement, l'organe de filtrage se présente sous la forme d'un filtre mécanique, en particulier un filtre poreux qui absorbe et retient les impuretés, tel qu'un filtre à charbon actif, ou sous la forme d'un filtre chimique qui détruit les molécules des impuretés, tel qu'un filtre par photocatalyse ou par ozonolise, ces deux types de filtre étant efficaces pour les types d'impuretés présentes dans l'habitacle.

L'invention concerne également un procédé de nettoyage de l'habitacle du véhicule de l'invention, comprenant : une étape de mesure du niveau d'insalubrité N de l'habitacle au moyen de l'organe de mesure, une étape d'activation de l'étape de chauffage lorsque le niveau d'insalubrité mesuré N est supérieur à un niveau d'insalubrité maximal prédéterminé N_{ET} au moyen de l'organe de commande, une étape de chauffage de l'habitacle par activation de l'organe de chauffage afin de chauffer l'air ambiant de l'habitacle et l'élément d'habitacle à une température supérieure à 40°C et ainsi de désorber les impuretés présentes et une étape de ventilation de l'habitacle afin d'évacuer l'air ambiant et les impuretés consommées à l'extérieur de l'habitacle au moyen de l'organe de ventilation.

De manière avantageuse, ce procédé permet de nettoyer l'habitacle d'un véhicule sans le déplacer vers un centre de nettoyage, l'ensemble du système de nettoyage requis pour effectuer le procédé étant interne au véhicule. Par ailleurs, ce procédé est autonome si bien qu'il ne requiert pas de main d'oeuvre. En outre, ce procédé permet de détecter le moment favorable pour effectuer le nettoyage, évitant un nettoyage anticipé inutile et un nettoyage tardif source de mécontentement des occupants.

De manière préférée, l'étape de chauffage de ce procédé est mise en oeuvre pendant une durée adaptée au niveau d'insalubrité N et au volume de l'habitacle de sorte à désorber les impuretés et à minimiser la consommation d'énergie.

Préférentiellement, la durée de l'étape de chauffage est adaptée pour désorber l'ensemble des impuretés tout en minimisant la consommation d'énergie.

Préférentiellement, au cours de l'étape d'activation, l'organe de commande active l'étape de chauffage lorsqu'il n'y a plus d'utilisateurs dans l'habitacle (état NOP), par mesure de sécurité et de confort pour les occupants (risque d'inconfort, de brûlure, mauvaises odeurs, etc.)

Préférentiellement, l'organe de commande active l'étape de chauffage lorsque l'habitacle est clos (état CLOS), c'est-à-dire que les portières et les vitres sont fermées, afin d'améliorer l'efficacité du procédé.

De manière préférée, l'étape de ventilation est mise en oeuvre jusqu'à ce que le niveau d'insalubrité N soit inférieur au niveau d'insalubrité maximal prédéterminé N_{ET}. De manière avantageuse, l'étape de ventilation est optimisée en matière de coût en temps et en énergie pour évacuer les impuretés.

De manière préférée, l'étape de ventilation est effectuée une fois l'étape de chauffage terminée et ce afin d'éviter les déperditions de chaleur.

Selon un aspect de l'invention, le procédé de nettoyage de l'habitacle du véhicule comprend une étape de filtrage des impuretés consommées au moyen de l'organe de filtrage. De manière avantageuse, cette étape permet d'éviter de rejeter de potentielles impuretés nocives dans l'atmosphère.

Préférentiellement, l'étape de filtrage est mise en oeuvre lors de l'étape de ventilation par gain de temps.

### PRESENTATION DES FIGURES

L'invention sera mieux comprise à la lecture de la description qui va suivre, donnée uniquement à titre d'exemple, et se référant aux dessins annexés donnés à titre d'exemples non limitatifs, dans lesquels des références identiques sont données à des objets semblables et sur lesquels :
La figure 1 est une représentation schématique de profil d'un véhicule automobile selon l'invention,
La figure 2 est une représentation schématique de dessus d'un véhicule automobile selon l'invention,
La figure 3 est une représentation schématique fonctionnelle du système de nettoyage d'habitacle de véhicule selon l'invention,
La figure 4 est une représentation schématique d'un organe de chauffage du système de nettoyage et
La figure 5 est une représentation schématique des étapes du procédé de nettoyage d'habitacle de véhicule selon l'invention.

Il faut noter que les figures exposent l'invention de manière détaillée pour mettre en oeuvre l'invention, lesdites figures pouvant bien entendu servir à mieux définir l'invention le cas échéant.

### DESCRIPTION DETAILLEE DE L'INVENTION

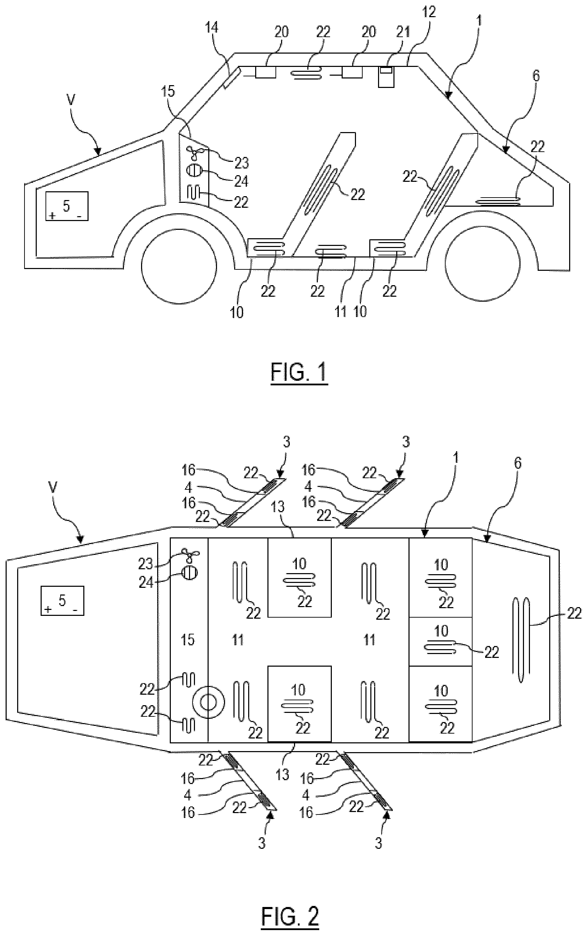

En référence aux figures 1 à 5, il est représenté de manière schématique un système et un procédé de nettoyage d'habitacle d'un véhicule automobile V par chauffage interne selon une forme de réalisation de l'invention. Comme indiqué précédemment, bien que l'invention soit née à l'origine pour un véhicule automobile, elle s'applique plus généralement à tout véhicule comportant un habitacle, en particulier, un train, un avion, un bateau, etc.

De manière connue et comme illustré sur les figures 1 et 2, un véhicule automobile V comprend un habitacle 1 défini comme la partie du véhicule V réservée aux utilisateurs, c'est-à-dire, l'espace de vie. Le véhicule automobile V comporte une pluralité de portières 3 permettant aux utilisateurs de monter et de descendre du véhicule V, c'est-à-dire d'accéder à l'habitacle 1, une pluralité de vitres 4 permettant de faire circuler un flux d'air entre l'intérieur et l'extérieur de l'habitacle 1 et un coffre 6 défini comme un espace de stockage.

Dans cet exemple, l'habitacle 1 comporte une pluralité de sièges 10, en particulier, deux sièges avant et une banquette arrière. L'habitacle 1 comprend en outre différents revêtements d'habitacle pour protéger et habiller l'habitacle 1, en particulier, un revêtement de plancher 11, un revêtement de pavillon 12 et un revêtement de parois latérales 13. De manière préférée, un revêtement 16 est également présent sur chaque portière 3. De tels revêtements permettent également d'assurer l'isolation thermique et sonore dans l'habitacle 1.

L'habitacle 1 comprend également une pluralité de pare-soleils 14 permettant aux utilisateurs de ne pas être éblouis en cas de forte luminosité extérieure, une planche de bord 15 située à l'avant du véhicule automobile 1 accueillant divers afficheurs et actionneurs pour commander le multimédia, la navigation, la ventilation et analogue. Dans cet exemple, le véhicule automobile V comporte en outre un espace de stockage connu sous le nom de « boîte à gants ».

De manière connue et comme illustré sur les figures 1 et 2, le véhicule automobile V comprend une batterie électrique 5 permettant de générer du courant électrique et ainsi alimenter les différents équipements électriques du véhicule V, à savoir les ampoules intérieures et extérieures, la radio ou encore les prises électriques à titre d'exemple.

Selon l'invention, le véhicule automobile V comporte un système de nettoyage 2 configuré pour retirer les impuretés du véhicule automobile V.

Grâce au système de nettoyage 2, le nettoyage du véhicule V est réalisé de manière interne, ce qui ne nécessite ni déplacement du véhicule dans un centre de nettoyage, ni dispositif extérieur à installer.

De manière avantageuse et comme illustré sur les figures 1 et 2, le système de nettoyage 2 est intégré dans l'habitacle 1 afin d'être au plus près des impuretés à nettoyer. En outre, cela permet de faciliter l'installation du système de nettoyage 2 ainsi que sa maintenance. Néanmoins, il va de soi qu'une partie du système de nettoyage 2 pourrait ne pas être comprise directement dans l'habitacle 1 mais positionnée à un autre endroit du véhicule automobile V, en particulier, dans un coffre arrière ou à proximité du moteur du véhicule automobile V.

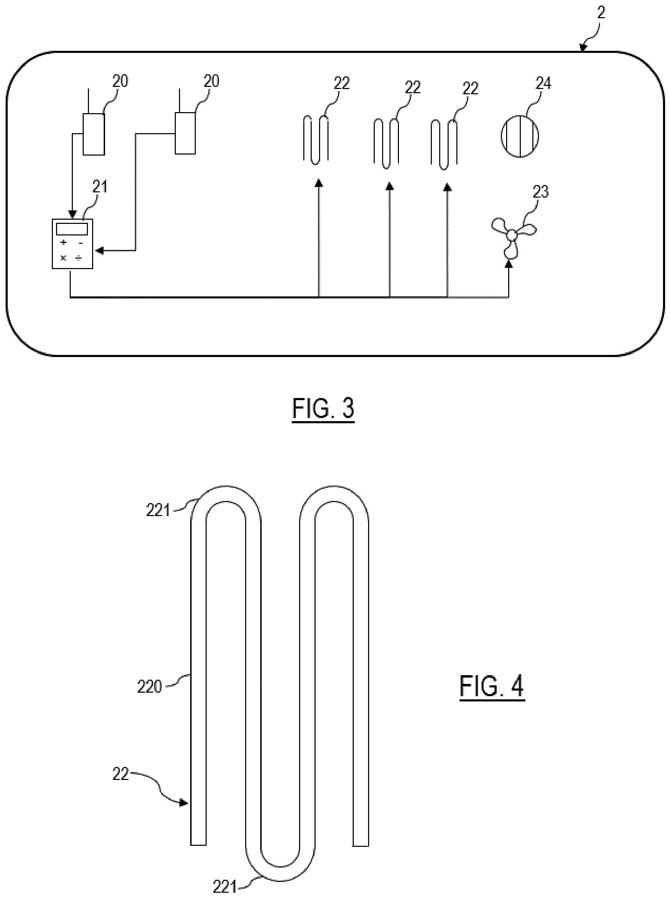

Dans cet exemple, en référence à la figure 3, le système de nettoyage 2 comprend une pluralité d'organes de mesure 20, une pluralité d'organes de chauffage 22, un organe de filtrage 24, un organe de ventilation 23 et un organe de commande 21 dont la structure et le rôle sont décrits successivement par la suite. Dans cet exemple, les différents organes 20-24 sont logés dans l'habitacle 1 et les différents organes 20-23 sont alimentés électriquement par la batterie électrique 5. Il va de soi que les organes 20-23 pourraient être alimentés de manière différente.

Comme illustré sur la figure 3, les organes de mesure 20 permettent de mesurer un niveau d'insalubrité N de l'habitacle 1. De manière avantageuse, ces organes de mesure 20 se présentent sous la forme de capteurs de gaz, tels que des capteurs à oxyde métallique à titre d'exemple, relevant la présence de toxiques volatils, qui sont le dioxyde d'azote N_{1,} le benzène toluène xylène N₂, l'ozone N₃ et le formaldéhyde N₄ ce qui permet de déterminer un niveau d'insalubrité N de l'habitacle 1. Il va de soi que d'autres types de capteurs pourraient convenir, en particulier, pour relever la présence de toxiques volatils non mentionnés, tels que des COV. De manière avantageuse, l'habitacle 1 comprend une pluralité d'organes de mesure 20, ce qui augmente la fiabilité et la précision de la mesure du niveau d'insalubrité N. Néanmoins, il va de soi que l'habitacle 1 pourrait ne contenir qu'un unique organe de mesure 20. Par ailleurs, de manière avantageuse, les organes de mesure 20 sont situés à l'avant de l'habitacle 1 à proximité de la position du conducteur afin de mesurer l'insalubrité ressentie par le conducteur. De manière préférée, en référence à la figure 1, les organes de mesure 20 sont répartis dans l'habitacle 1 afin de mesurer une moyenne représentative du niveau d'insalubrité global et non un niveau d'insalubrité local d'une zone de l'habitacle 1.

Selon l'invention, les organes de chauffage 22 permettent, en service, de produire de la chaleur, qui se transmet aux éléments d'habitacle 10-16 par conduction et à l'air ambiant par convection. La température dans l'habitacle 1 augmente alors ce qui a pour effet de désorber les impuretés présentes, c'est-à-dire d'extraire les impuretés des éléments de l'habitacle et de les faire passer dans une phase vapeur.

Les organes de chauffage 22 sont configurés pour chauffer l'air ambiant de l'habitacle 1 à une température supérieure à 40°C, de préférence supérieure à 45°C, de préférence encore à 50°C, afin de désorber les impuretés de l'habitacle 1. De préférence, la température de chauffage est inférieure à 60°C de manière à ne pas dégrader des éléments d'habitacle 1 comme cela va être présenté par la suite.

Dans cet exemple, en référence à la figure 4, chaque organe de chauffage 22 se présente sous la forme d'une résistance en serpentin planaire. Autrement dit, chaque organe de chauffage 22 comprend une alternance de tubes cylindriques rectilignes 220 et de tubes cylindriques coudés 221. Un tel organe de chauffage 22 permet de chauffer de manière homogène une surface donnée. Il va de soi que les organes de chauffage 22 pourraient posséder une forme différente.

Selon un aspect de l'invention, les organes de chauffage 22 sont alimentés de manière filaire ou sans fil, en particulier, par induction. Une alimentation sans fil permet avantageusement de rendre l'élément d'habitacle amovible de l'habitacle, par exemple, un tapis de sol. Ces deux modes de chauffage permettent de désorber efficacement les impuretés. Il va cependant de soi qu'un autre mode de chauffage peut être choisi.

Selon un autre aspect de l'invention, les organes de chauffage 22 peuvent être rigides ou souples, le choix étant basé sur leur intégration dans l'habitacle 1.

Selon un aspect de l'invention, plusieurs éléments d'habitacle 10-16 sont équipés d'un organe de chauffage 22 pour former un élément d'habitacle chauffant et ce afin de couvrir le plus grand volume possible de l'habitacle 1 sujet à s'imprégner d'impuretés. Il va toutefois de soi que seul un ou une partie des éléments d'habitacle 10-16 peuvent être équipés d'un organe de chauffage 22, et de manière complémentaire, qu'un même élément d'habitacle 10-16 peut être équipé d'une pluralité d'organes de chauffage 22. Des éléments d'habitacle 10-16 non cités explicitement, par souci de concision et de clarté, peuvent également être équipés d'un ou plusieurs organes de chauffage 22. En outre, chaque organe de chauffage 22 peut être installé de deux façons différentes suivant la commodité d'installation et suivant l'usage de l'élément d'habitacle 10-16, à savoir par insertion/intégration dans ledit élément ou tapissant en surface ledit élément. L'association d'un organe de chauffage 22 à un élément d'habitacle 10-16 est avantageuse étant donné qu'elle permet de chauffer l'air ambiant de l'habitacle 1 mais également de chauffer de manière interne l'élément d'habitacle 10-16 auquel est associé l'organe de chauffage 22 afin de désorber thermiquement les impuretés contenues dans ledit élément d'habitacle 10-16.

A titre d'exemple, comme illustré sur les figures 1 et 2, au moins un organe de chauffage 22 est inséré dans chaque siège 10, dans chaque pare-soleil 14 ainsi que dans la planche de bord 15. Des organes de chauffage 22 tapissent également le revêtement du plancher 11, le revêtement du pavillon 12, les parois latérales 13 et les revêtements 16 de portière 3. Dans cet exemple, une grande partie des éléments d'habitacle 10-16 est ainsi équipée d'organes de chauffage 22 et forme des éléments d'habitacle chauffants. Pour les éléments d'habitacle 10-16 destinés au confort, par exemple un siège 10, il est préférable d'intégrer l'organe de chauffage 22 dans le siège 10 plutôt que de le tapisser afin de ne pas nuire au confort des utilisateurs. Au contraire, pour le revêtement de plancher 11, l'organe de chauffage 2 peut être placé en surface pour réduire le coût.

En outre, dans l'exemple du paragraphe précédent, le coffre 6 est tapissé d'un organe de chauffage 22, bien qu'externe à l'habitacle 1, car néanmoins susceptible d'être souillé par les utilisateurs au même titre que les éléments d'habitacle 10-16. Par exemple, le coffre 6 peut être sali par les bagages des utilisateurs, par des fuites de liquides ou par un animal de compagnie.

Par ailleurs, les organes de chauffage 22 sont intégrés dans des éléments d'habitacle 10-16 comprenant de la mousse polyuréthane ou du polychlorure de vinyle ou de l'acrylonitrile butadiène styrène ou du polypropylène ou du polyéthylène ou du polyester ou un alliage de ces thermoplastiques, de sorte à améliorer le chauffage et la désorption. Il va toutefois de soi que les éléments d'habitacle 10-16 peuvent comprendre d'autres matériaux.

Comme indiqué précédemment, l'organe de chauffage 22 est configuré pour chauffer l'air ambiant de l'habitacle 1 à une température supérieure à 40°C. Autrement dit, la température extérieure de l'élément d'habitacle 10-16 est supérieure à 40°C. Cela se distingue d'un siège chauffant conventionnel dont la température extérieure est de l'ordre de 30°C et inférieure à 40°C.

L'organe de ventilation 23 permet d'évacuer l'air ambiant et les impuretés désorbées à l'extérieur de l'habitacle 1. Dans cet exemple, il est présenté un unique organe de ventilation 23 mais il va de soi qu'une pluralité d'organes de ventilation 23 pourrait être utilisée. Préférentiellement, l'organe de ventilation 23 est situé au niveau de la planche de bord afin de tirer parti des conduites de ventilation existantes. De préférence, l'organe de ventilation 23 se présente sous la forme d'un ventilateur mécanique.

L'organe de filtrage 24 permet de traiter, de préférence mécaniquement ou chimiquement, les impuretés désorbées avant qu'elles ne soient évacuées à l'extérieur de l'habitacle 1, par souci écologique. Un tel organe de filtrage 24 est optionnel. Préférentiellement, l'organe de filtrage 24 est associé à l'organe de ventilation 23 afin de traiter et évacuer les impuretés désorbées simultanément mais il va de soi que l'organe de filtrage 24 et l'organe de ventilation 23 peuvent être distincts. De préférence, l'organe de filtrage 24 se présente sous la forme d'un filtre chimique qui détruit les molécules des impuretés, par exemple un filtre par ozonolize ou par photocatalyse, ou encore sous la forme d'un filtre mécanique, par exemple un filtre à charbon actif. De préférence, l'organe de filtrage 24 est placé en entrée de l'organe de ventilation 23.

L'organe de commande 21 est configuré pour activer les organes de chauffage 22 lorsque le niveau d'insalubrité N de l'habitacle 1 est supérieur à un niveau d'insalubrité maximal prédéterminé N_{ET}, c'est-à-dire lorsque le véhicule est dans un état sale POK. De manière avantageuse, le niveau d'insalubrité maximal N_{ET} est déterminé à partir de banques de données sur la présence maximale autorisée de toxiques volatils dans l'habitacle 1 du véhicule V, afin d'adopter une échelle de comparaison la plus pertinente possible et adaptée au cas du véhicule V considéré. Toutefois, le niveau d'insalubrité maximal N_{ET} peut être déterminé de manière différente. Dans cet exemple, l'organe de commande 21 se présente sous la forme d'un calculateur électronique relié électriquement aux organes de mesure 20, aux organes de chauffage 22 et à l'organe de ventilation 23 afin de commander les étapes de nettoyage.

II va être dorénavant présenté un exemple de mise en oeuvre d'un procédé de nettoyage du véhicule V selon l'invention.

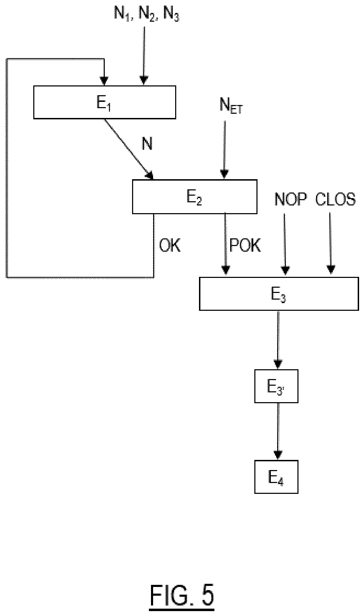

En référence à la figure 5, le procédé de nettoyage de l'habitacle 1 du véhicule V comprend une étape de mesure E₁ du niveau d'insalubrité N de l'habitacle 1 au moyen des organes de mesure 20, une étape d'activation E₂, par l'organe de commande 21, des organes de chauffage 22 afin de réaliser une étape de chauffage E₃ à une température supérieure à 40°C lorsque le niveau d'insalubrité mesuré N est supérieur à un niveau d'insalubrité maximal prédéterminé N_{ET}.

De préférence, lors de l'étape de mesure E₁, les organes de mesure 20 relèvent, à différents instants, le taux de dioxyde d'azote N₁, de benzène toluène xylène N₂, d'ozone N₃ et de formaldéhyde N₄ dans l'habitacle 1 afin de définir le niveau d'insalubrité N dudit habitacle 1. Il va de soi que les organes de mesure 20 pourraient mesurer d'autres types d'impuretés tels que des COV. De préférence, l'étape de mesure E₁ est mise en oeuvre de manière périodique ou à chaque fois que le véhicule automobile V est inactivé.

Lors de l'étape d'activation E₂, l'organe de commande 21 compare le niveau d'insalubrité N mesuré de l'habitacle 1 à différents instants au niveau d'insalubrité maximal prédéterminé N_{ET} et permet de déterminer avec précision quand procéder à l'étape de chauffage E₃. Ainsi, l'étape de chauffage est activée lorsque l'habitacle 1 est considéré comme étant à l'état sale POK, à savoir lorsque le niveau d'insalubrité mesuré N est supérieur au niveau d'insalubrité maximal prédéterminé N_{ET}. Lorsque le niveau d'insalubrité mesuré N est inférieur au niveau d'insalubrité maximal prédéterminé N_{ET}, l'habitacle 1 est considéré comme étant à l'état propre OK et aucun chauffage n'est réalisé.

Selon un aspect de l'invention, l'étape de chauffage E₃ est activée uniquement lorsque l'habitacle 1 est clos (état CLOS). A cet effet, le système de chauffage 2 comporte un ou plusieurs capteurs de fermeture (non représentés), positionnés au niveau des portières 3 ou des vitres 4, afin de déterminer si l'habitacle 1 est clos. De préférence, les capteurs de fermeture sont reliés électriquement à l'organe de commande 21.

Selon un autre aspect de l'invention, l'étape de chauffage E₃ est activée uniquement en l'absence d'utilisateurs (état NOP). A cet effet, le système de chauffage 2 comporte un ou plusieurs capteurs de présence (non représentés), positionnés dans l'habitacle 1, afin de déterminer si des utilisateurs sont présents. De préférence, les capteurs de présence sont reliés électriquement à l'organe de commande 21. L'étape de chauffage E₃ permet ainsi un traitement efficace, c'est-à-dire sans déperdition de chaleur et sans désagrément pour les utilisateurs du véhicule.

L'étape de chauffage E₃ est mise en oeuvre par activation des organes de chauffage 22 de sorte que l'air ambiant soit à une température supérieure à 40°C, de préférence supérieure à 45°C, de préférence encore à 50°C, afin de désorber l'ensemble des impuretés présentes et donc de permettre un nettoyage efficace et non partiel. De préférence, la température des organes de chauffage 22 n'excède pas 60°C et ce afin de ne pas altérer les éléments d'habitacle 10-16. Durant cette étape, les éléments d'habitacle chauffants sont donc purifiés de leurs impuretés par la chaleur dégagée par leur organe de chauffage 22 transmise par conduction. L'air ambiant est quant à lui également purifié par convection de la chaleur des éléments d'habitacle chauffants. Ce procédé permet donc de purifier avantageusement les éléments d'habitacle chauffants et l'air ambiant de leurs impuretés, c'est-à-dire l'habitacle 1 dans son ensemble, ce qui rend le procédé très efficace.

De manière préférée, l'étape de chauffage E₃ est mise en oeuvre pendant une durée adaptée au niveau d'insalubrité N et au volume de l'habitacle 1 de sorte à désorber au moins 70% des impuretés et à minimiser la consommation d'énergie. De manière préférée, la durée est telle que l'ensemble des impuretés sont désorbées.

En référence à la figure 5, le procédé comporte une étape de filtrage E_{3'} des impuretés consommées par l'organe de filtrage 24 et une étape de ventilation E₄ de l'habitacle 1 par l'organe de ventilation 23 afin d'évacuer l'air ambiant et les impuretés désorbées à l'extérieur de l'habitacle 1 au moyen de l'organe de ventilation 23.

L'étape de filtrage E_{3'} est mise en oeuvre en traitant les impuretés désorbées afin de leur retirer d'éventuels composants nocifs. Cette étape n'est pas indispensable au procédé de nettoyage de l'habitacle 1 du véhicule V mais est réalisée de manière avantageuse par souci écologique pour éviter la propagation des impuretés désorbées potentiellement nocives dans l'atmosphère. Cette étape est réalisée une fois l'étape de chauffage E₃ terminée ou de manière simultanée lors que la ventilation est réalisée sur un circuit fermé.

L'étape de ventilation E₄ est mise en oeuvre en mettant en marche l'organe de ventilation 23 jusqu'à ce que le niveau d'insalubrité N de l'habitacle 1 soit inférieur au niveau d'insalubrité maximal prédéterminé N_{ET}. De préférence, l'organe de ventilation 23 fonctionne à une puissance adaptée au niveau d'insalubrité N et au volume de l'habitacle de sorte à évacuer rapidement les impuretés en suspension dans l'air ambiant à l'extérieur de l'habitacle 1 via l'organe de ventilation 23, tout en minimisant la consommation d'énergie. Selon un aspect de l'invention, cette étape de ventilation E₄ est opérée une fois l'étape de chauffage E₃ terminée afin d'éviter des déperditions de chaleur et lors de l'étape de filtrage E_{3'}, les impuretés désorbées étant ainsi traitées au moment d'être évacuées à l'extérieur. Cependant, il va de soi que cette étape de ventilation E₄ peut être lancée parallèlement à l'étape de chauffage E₂ et après l'étape de filtrage E_{3'}. De manière avantageuse, cette étape est rapide et facile à réaliser et utilise des moyens internes au véhicule V.

De préférence, en l'absence d'étape de filtrage E_{3'}, les vitres 4 du véhicule automobile V sont ouvertes lors de l'étape de ventilation E₄.

De manière avantageuse, ce procédé permet de déterminer avec précision quand procéder au nettoyage de l'habitacle 1, évitant ainsi un gaspillage de temps et d'argent lié à un nettoyage trop anticipé et le mécontentement des utilisateurs du véhicule V lié à un nettoyage trop tardif. Ce procédé présente également l'avantage de ne pas nécessiter de déplacement de véhicule ni de main d'oeuvre pour être réalisé, étant interne au véhicule V, ce qui le rend moins onéreux. Un tel procédé de nettoyage est parfaitement adapté à un véhicule ayant un taux d'occupation élevé.

## Revendications

1. Véhicule (V) comprenant un habitacle (1), véhicule (V) comprenant un système de nettoyage (2) de l'habitacle (1) comprenant au moins un organe de mesure (20) du niveau d'insalubrité (N) dudit habitacle (1), au moins un organe de chauffage (22) configuré pour chauffer l'air ambiant de l'habitacle (1) à une température supérieure à 40°C afin de désorber les impuretés de l'habitacle (1), et au moins un organe de commande (21) configuré pour activer l'organe de chauffage (22) si le niveau d'insalubrité (N) mesuré est supérieur à un niveau d'insalubrité maximal prédéterminé (N_{ET}), **caractérisé par le fait que** le véhicule comprend au moins un élément d'habitacle (10-16) comprenant au moins un organe de chauffage (22) configuré pour chauffer l'élément d'habitacle (10-16) à une température supérieure à 40°C afin de former un élément d'habitacle chauffant pour désorber les impuretés de l'élément d'habitacle (10-16) et au moins un organe de ventilation (23) configuré pour évacuer l'air ambiant et les impuretés désorbées à l'extérieur de l'habitacle (1).

2. Véhicule (V) selon la revendication 1, dans lequel l'organe de chauffage (22) est configuré pour chauffer l'air ambiant de l'habitacle (1) à une température supérieure à 45°C, de préférence 50°C.

3. Véhicule (V) selon l'une des revendications 1 et 2, dans lequel l'organe de chauffage (22) est configuré pour chauffer l'air ambiant de l'habitacle (1) à une température inférieure à 60°C.

4. Véhicule (V) selon l'une des revendications 1 à 3, dans lequel l'organe de chauffage (22) est intégré de manière interne à l'élément d'habitacle (10-16).

5. Véhicule (V) selon l'une des revendications 1 à 3, dans lequel l'organe de chauffage (22) est positionné en surface de l'élément d'habitacle (10-16).

6. Véhicule (V), selon l'une des revendications 1 à 5, dans lequel au moins un élément d'habitacle chauffant est un siège (10).

7. Véhicule (V), selon l'une des revendications 1 à 6, dans lequel au moins un élément d'habitacle chauffant est un revêtement de l'habitacle (1), en particulier, un revêtement de plancher (11).

8. Véhicule (V) selon l'une des revendications 1 à 7, dans lequel au moins un élément d'habitacle chauffant est la planche de bord.

9. Véhicule (V), selon l'une des revendications 1 à 8, dans lequel le système de nettoyage (2) comprend au moins un organe de filtrage (24) configuré pour traiter les impuretés désorbées lors de leur évacuation vers l'extérieur de l'habitacle (1).

10. Procédé de nettoyage de l'habitacle (1) d'un véhicule (V) selon l'une des revendications 1 à 9, comprenant :
- une étape de mesure (E₁) du niveau d'insalubrité (N) de l'habitacle (1) au moyen de l'organe de mesure (20),
- une étape d'activation (E₂) de l'étape de chauffage (E₃) lorsque le niveau d'insalubrité mesuré (N) est supérieur à un niveau d'insalubrité maximal prédéterminé (N_{ET}) au moyen de l'organe de commande (21),
- une étape de chauffage (E₃) de l'habitacle (1) par activation de l'organe de chauffage (22) afin de chauffer l'air ambiant de l'habitacle (1) et l'élément d'habitacle (10-16) à une température supérieure à 40°C et ainsi de désorber les impuretés présentes et
- une étape de ventilation (E₄) de l'habitacle (1) afin d'évacuer l'air ambiant et les impuretés désorbées à l'extérieur de l'habitacle (1) au moyen de l'organe de ventilation (23).

## Patentansprüche

1. Fahrzeug (V), das einen Innenraum (1) umfasst, wobei das Fahrzeug (V) ein Reinigungssystem (2) des Innenraums (1) umfasst, umfassend mindestens ein Messorgan (20) des Verschmutzungsgrads (N) des Innenraums (1), mindestens ein Heizorgan (22), das ausgelegt ist, um die Raumluft des Innenraums (1) auf eine Temperatur über 40 °C zu erwärmen und die Verschmutzungen des Innenraums (1) zu desorbieren, und mindestens ein Steuerorgan (21), das ausgelegt ist, um das Heizorgan (22) zu aktivieren, wenn der gemessene Verschmutzungsgrad (N) über einem vorher festgelegten maximalen Verschmutzungsgrad (N_{ET}) liegt, **dadurch gekennzeichnet, dass** das Fahrzeug (V) mindestens ein Innenraumelement (10-16) umfasst, das mindestens ein Heizorgan (22) umfasst, das ausgelegt ist, um das Innenraumelement (10-16) auf eine Temperatur über 40 °C zu erwärmen, um ein Heiz-Innenraumelement zu bilden, um die Verschmutzungen vom Innenraumelement (10-16) zu desorbieren, und mindestens ein Lüftungsorgan (23), das ausgelegt ist, um die Raumluft und die desorbierten Verschmutzungen aus dem Innenraum (1) abzuleiten.

2. Fahrzeug (V) nach Anspruch 1, wobei das Heizorgan (22) ausgelegt ist, um die Raumluft des Innenraums (1) auf eine Temperatur über 45 °C zu erwärmen, vorzugsweise über 50 °C.

3. Fahrzeug (V) nach einem der Ansprüche 1 und 2, wobei das Heizorgan (22) ausgelegt ist, um die Raumluft des Innenraums (1) auf eine Temperatur unter 60 °C zu erwärmen.

4. Fahrzeug (V) nach einem der Ansprüche 1 bis 3, wobei das Heizorgan (22) innen in das Innenraumelement (10-16) integriert ist.

5. Fahrzeug (V) nach einem der Ansprüche 1 bis 3, wobei das Heizorgan (22) an der Oberfläche des Innenraumelements (10-16) positioniert ist.

6. Fahrzeug (V), nach einem der Ansprüche 1 bis 5, wobei mindestens ein Heiz-Innenraumelement ein Sitz (10) ist.

7. Fahrzeug (V), nach einem der Ansprüche 1 bis 6, wobei mindestens ein Heiz-Innenraumelement eine Verkleidung des Innenraums (1), vor allem eine Bodenverkleidung (11) ist.

8. Fahrzeug (V) nach einem der Ansprüche 1 bis 7, wobei mindestens ein Heiz-Innenraumelement die Instrumententafel ist.

9. Fahrzeug (V) nach einem der Ansprüche 1 bis 8, wobei das Reinigungssystem (2) mindestens ein Filterorgan (24) umfasst, das ausgelegt ist, um die desorbierten Verschmutzungen bei ihrer Ableitung aus dem Innenraum (1) zu bearbeiten.

10. Verfahren zur Reinigung des Innenraums (1) eines Fahrzeugs (V) nach einem der Ansprüche 1 bis 9, umfassend:
- einen Messschritt (E₁) des Verschmutzungsgrads (N) des Innenraums (1) mittels des Messorgans (20),
- einen Aktivierungsschritt (E₂) des Heizschritts (E₃), wenn der gemessene Verschmutzungsgrad (N) über einem vorher festgelegten maximalen Verschmutzungsgrad (N_{ET}) liegt, mittels des Steuerorgans (21),
- einen Heizschritt (E₃) des Innenraums (1) durch Aktivierung des Heizorgans (22), um die Raumluft des Innenraums (1) zu erwärmen und so die vorhandenen Verschmutzungen zu desorbieren, und
- einen Lüftungsschritt (E₄) des Innenraums (1), um die Raumluft und die desorbierten Verschmutzungen aus dem Innenraum (1) mittels des Lüftungsorgans (23) abzuleiten.

## Claims

1. A vehicle (V) comprising a passenger compartment (1), the vehicle (V) comprising a system (2) for cleaning the passenger compartment (1) comprising at least one member (20) for measuring the level of insalubrity (N) of said passenger compartment (1), at least one heating member (22) configured to heat the ambient air of the passenger compartment (1) to a temperature greater than 40 °C in order to desorb the impurities from the passenger compartment (1), and at least one control member (21) configured to activate the heating member (22) if the level of insalubrity (N) measured is greater than a predetermined maximum level of insalubrity (N_{ET}), **characterised by the fact** that the vehicle comprises at least one passenger compartment element (10-16) comprising at least one heating member (22) configured to heat the passenger compartment element (10-16) to a temperature greater than 40 °C in order to form a heated passenger compartment element to desorb the impurities from the passenger compartment element (10-16) and at least one ventilation member (23) configured to discharge the ambient air and impurities desorbed outside the passenger compartment (1).

2. The vehicle (V) according to claim 1, wherein the heating member (22) is configured to heat the ambient air of the passenger compartment (1) to a temperature greater than 45 °C, preferably 50 °C.

3. The vehicle (V) according to one of claims 1 to 2, wherein the heating member (22) is configured to heat the ambient air of the passenger compartment (1) to a temperature lower than 60 °C.

4. The vehicle (V) according to one of claims 1 to 3, wherein the heating member (22) is internally integrated into the passenger compartment element (10-16).

5. The vehicle (V) according to one of claims 1 to 3, wherein the heating member (22) is positioned on the surface of the passenger compartment element (10-16).

6. The vehicle (V), according to one of claims 1 to 5, wherein at least one heated passenger compartment element is a seat (10).

7. The vehicle (V), according to one of claims 1 to 6, wherein at least one heated passenger compartment element is a covering of the passenger compartment (1), in particular, a floor covering (11).

8. The vehicle (V) according to one of claims 1 to 7, wherein at least one heated passenger compartment element is the dashboard.

9. The vehicle (V), according to one of claims 1 to 8, wherein the cleaning system (2) comprises at least one filtering member (24) configured to treat the impurities desorbed when they are discharged to the outside of the passenger compartment (1).

10. A method for cleaning the passenger compartment (1) of a vehicle (V) according to one of claims 1 to 9, comprising:
- a step (E₁) of measuring the level of insalubrity (N) of the passenger compartment (1) using the measuring member (20),
- a step (E₂) of activating the heating step (E₃) when the level of insalubrity (N) measured is greater than a predetermined maximum level of insalubrity (N_{ET}) by means of the control member (21),
- a step (E₃) of heating the passenger compartment (1) by activating the heating element (22) in order to heat the ambient air of the passenger compartment (1) and the passenger compartment element (10-16) to a temperature greater than 40 °C and thus desorb the impurities present, and
- a step (E₄) of ventilating the passenger compartment (1) in order to discharge the ambient air and impurities desorbed outside the passenger compartment (1) by means of the ventilation member (23).
